(19) Europäisches Patentamt
European Patent Office
Office européen des brevets

(11) Publication number: **0 337 019 A1**

# (12) EUROPEAN PATENT APPLICATION

(21) Application number: 88303239.3

(22) Date of filing: 12.04.88

(51) Int. Cl.[4]: C07D 405/06 , A61K 31/415 , //C07D407/12,C07D405/12

(43) Date of publication of application: 18.10.89 Bulletin 89/42

(84) Designated Contracting States: ES GR

(71) Applicant: SCHERING CORPORATION
2000 Galloping Hill Road
Kenilworth New Jersey 07033(US)

(72) Inventor: Lovey, Raymond G.
64 Woodside Avenue
West Caldwell New Jersey 07006(US)
Inventor: Elliott, Arthur J.
62 Seven Lakes Drive
Sloatsburg New York 10974(US)

(74) Representative: Ritter, Stephen David et al
Mathys & Squire 10 Fleet Street
London EC4Y 1AY(GB)

## (54) Novel 1-aryl-1-(1H-imidazol-1'-yl methyl)-1,3-dihydroisobenzofurans, their preparation and pharmaceutical compositions containing them.

(57) Disclosed herein are novel 1-aryl-1-(1H-imidazol-1-ylmethyl)-1,3-dihydrobenzofurans of the general formula I:

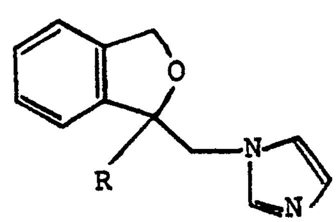

(I),

wherein R represents a halo- and/or trifluoromethyl-substituted phenyl group, the compounds exhibiting useful in vivo antifungal activity. Also disclosed are processes for the preparation of the disclosed compounds as well as pharmaceutical compositions containing them.

EP 0 337 019 A1

# NOVEL-1-ARYL-1-(1H-IMIDAZOL-1-YLMETHYL)-1,3-DIHYDROISOBENZOFURANS, THEIR PREPARATION AND PHARMACEUTICAL COMPOSITIONS CONTAINING THEM

This invention relates to novel 1-aryl-1-(1H-imidazol-1-ylmethyl)-1,3-dihydroisobenzofurans, their preparation and pharmaceutical compositions containing them, the novel compounds possessing valuable antifungal activity against human and animal pathogens.

In British Patent Application 2,143,523A there is disclosed a group of 1-aryl-1-(1,2,4-triazol-1-ylmethyl and imidazol-1-ylmethyl)-1,3-dihydroisobenzofurans for use as plant growth regulating agents and for use in treating fungal diseases in plants. At page 6, line 45 of the specification, a reference occurs to the compounds being useful in treating human fungal infections.

We have now found that a narrow group of compounds falling within the group of compounds generally disclosed in G.B. 2,143,523A possesses unexpectedly valuable *in vivo* antifungal activity against human and animal pathogens including dermatophytes and strains of *Candida*.

Accordingly, in one of its aspects the present invention provides novel compounds of the general formula I:

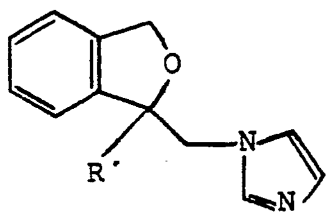

(I),

and the pharmaceutically acceptable salts thereof, wherein R represents a halo- and/or trifluoromethyl-substituted phenyl group. Representative halo-substituents are bromo and in particular chloro and fluoro. The phenyl group may suitably be mono-, di- or tri-substituted by the halo and/or trifluoromethyl substituents, the substituents being the same or different. Preferred are mono-substituted phenyl and in particular di-substituted phenyl groups. Representative monohalo-substituted phenyl groups are 2-, 3- and 4-chlorophenyl and the corresponding fluoro substituted phenyl groups; representative of the preferred dihalosubstituted-phenyl groups are 2,4-, 2,5- and 2,6-dichlorophenyl and the corresponding fluoro substituted phenyl groups.

The compounds of the general formula I may exist in isomeric forms and the present invention includes the racemic mixtures and the individual isomers.

Typical pharmaceutically acceptable salts of the compounds of the general formula I are pharmaceutically acceptable acid addition salts derived from mineral acids such as HCl, HBr, $HNO_3$, $H_2SO_4$ or $H_3PO_4$ or from an organic acid, such as para-toluene sulfonic, methane sulfonic, maleic, and the like.

These salts may be obtained by employing conventional procedures such as, for example, by mixing solutions containing stoichiometric amounts of the free base of the general formula (I) and the desired acid together, followed by filtration to collect the required salt, if insoluble, or by evaporation of the solvent from the system in accordance with standard techniques.

In another of its aspects the present invention provides a process, herein identified as Process A, for the preparation of a compound of the general formula I hereinabove defined or a pharmaceutically acceptable salt thereof, which process comprises cyclizing a compound of the general formula II:

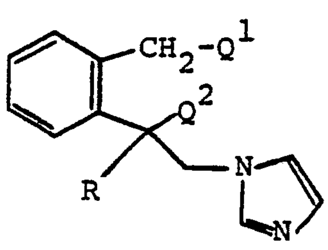

(II),

wherein R is as defined for formula I and of $Q^1$ and $Q^2$ one is hydroxy and the other is hydroxy, halo or a reactive ether or ester group, in the presence of a solvent and a cyclizing agent and at a temperature up to

the reflux temperature of the reaction mixture, and then isolating the so obtained compound of the general formula I in free base form or in the form of a pharmaceutically acceptable salt.

In the foregoing Process A, $Q^2$ can, for example, be hydroxy and $Q^1$ can be hydroxy or a reactive ether group such as acetoxy-methoxy, 2-methoxy-ethoxy-methoxy or, preferably, 2-tetrahydropyranyloxy and the cyclization effected in the presence of an acid as cyclizing agent, preferably a strong mineral acid such as sulfuric acid, or especially hydrochloric acid. The reaction may suitably be carried out in wholly aqueous medium or in an aqueous organic medium such as in aqueous ethanol or in an aqueous mixture of another water-miscible organic solvent such as dioxane. The reaction temperature may be a temperature up to the reflux temperature of the reaction mixture, preferably from about 60 °C up to the reflux temperature.

Alternatively, in Process A, $Q^2$ can be hydroxy and $Q^1$ can be halo, preferably chloro, or an acyloxy group of a strong organic acid, especially a sulfonic acid, preferably methane sulfonic or, in particular, p-toluene sulfonic acid. Cyclization may be effected by treating the starting material of the general formula II with a strong non-nucleophilic base, as cyclizing agent, in an inert organic solvent at a temperature up to the reflux temperature of the reaction mixture, preferably from room temperature up to the reflux temperature. Suitable cyclizing agents include alkali metal hydrides such as sodium hydride (typically employed in an inert organic solvent such as dimethylformamide), alkali metal alkoxides such as potassium butoxide (which may be employed for instance in tertiary butyl alcohol as the inert organic solvent) and alkali metal alkyls (which may be employed in tetrahydrofuran as the inert organic solvent).

The starting material of the general formula II for use in Process A may be prepared by methods known per se. Thus, for preparing a compound of the general formula II where $Q^2$ is hydroxy and $Q^1$ is a reactive ether group, for instance a 2-tetrahydropyranyloxy group, the following outline reaction scheme I may be employed where R is as defined for formula I and THP represents 2-tetrahydropyranyloxy:

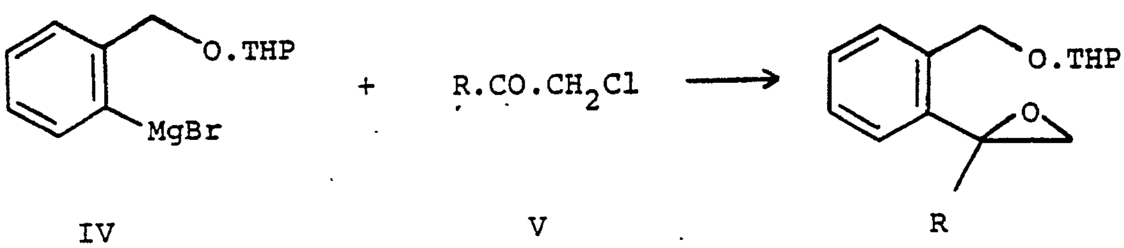

IV V VI

VI +

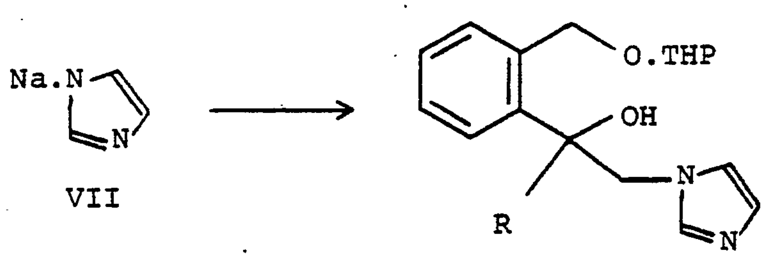

VII IIA

In the foregoing reaction scheme, the Grignard starting material IV, prepared in a known manner, is treated with an appropriately substituted phenacyl chloride V to yield the oxirane VI which is then reacted under appropriate conditions with an alkali metal salt of imidazole, such as the sodium salt, to give the desired starting material of the general formula IIA.

For preparing a starting material of the general formula II where $Q^1$ and $Q^2$ are both hydroxy, a compound of the general formula IIA may be subjected to controlled acid hydrolysis. If desired, the resulting product may then be treated with an appropriate halogenating agent, such as triphenyl phosphine in carbon tetrachloride, to give a starting material of the formula II wherein $Q^1$ is halo and $Q^2$ is hydroxy.

Alternatively, where a starting material is required of the general formula II wherein $Q^1$ is a reactive ester group and $Q^2$ is hydroxy, the corresponding diol may be reacted with an appropriate organic ester halide or anhydride, for instance for the preparation of a mesyl ester the corresponding diol may be reacted with mesyl chloride and triethylamine in dichloromethane.

In another of its aspects, the present invention provides a further process, herein identified as Process B, for the preparation of a compound of the general formula I hereinabove defined or a pharmaceutically

acceptable salt thereof, which process comprises reacting a compound of the general formula III:

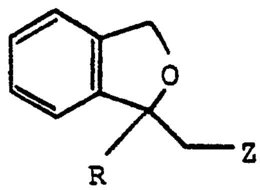

(III),

wherein R is as defined for formula I and Z is halo or is a reactive ester group, with imidazole in the presence of a strong base, or with an alkali metal derivative of imidazole, in an aprotic organic solvent at a temperature up to the reflux temperature of the reaction mixture.

In the compound of the general formula III, Z may suitably be bromo, iodo or preferably chloro, or an acyloxy group of a strong organic acid, especially a sulfonic acid, preferably methane sulfonic acid or p-toluene sulfonic acid. The strong base in Process B may suitably be an alkali metal hydride, e.g. NaH. Where an alkali metal derivative of imidazole is employed, that may suitably be the sodium salt. A suitable aprotic organic solvent is dimethyl formamide.

The starting material of the general formula III for Process B may be prepared from the oxirane of the general formula VI set forth in the description of Process A by the following outline reaction scheme:

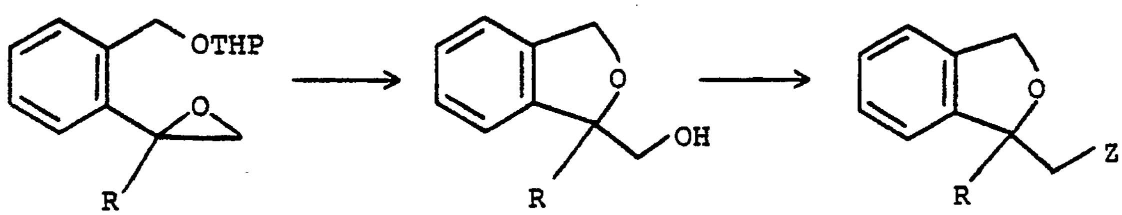

VI VIII III

The oxirane VI may be treated with a suitable organic or mineral acid in aqueous media to yield the compound of the general formula VIII which by treating with an appropriate halogenating agent or with an appropriate organic acid halide or anhydride (e.g. mesyl chloride) can be converted to the desired compound of the formula III where Z is halo or a reactive ester group.

The invention will now be illustrated by way of the following specific examples.

## EXAMPLE 1

### 1-(2,4-DICHLOROPHENYL)-1-(1H-IMIDAZOL-1-YLMETHYL)-1,3-DIHYDROISOBENZOFURAN HYDROCHLORIDE

#### A. 1-(2,4-DICHLOROPHENYL)-1-[2-[(2-TETRAHYDROPYRANYL)OXY]-METHYL]PHENYL OXIRANE

Prepare the Grignard reagent by adding 15.1g of 2-bromobenzyl-(2-tetrahydropyranyl)ether to 1.35g of magnesium in 30 mL of tetrahydrofuran (THF). Add the Grignard reagent so formed dropwise over a period of 15 minutes at 0-5° C to a solution of 11.0g of 2,4-dichlorophenacyl chloride in 150 mL of THF. Allow the solution to warm to room temperature, and stir for 18 hours. Cool the reaction mixture to 0° C. Vigorously stir the cooled reaction mixture and add 100 mL of saturated aqueous $NH_4Cl$. Separate the aqueous layer and extract with ethyl acetate (EtOAc), and wash the combined THF-EtOAc extracts with saturated brine and dry over anhydrous $Na_2SO_4$. Filter the mixture and evaporate the filtrate to leave 16g of crude 1-(2,4-dichlorophenyl)-1-[2-[(2-tetrahydropyranyl)oxy]methyl]phenyl oxirane, which is used in the next step without further purification.

B. 2-(1H-IMIDAZOL-1-YL)-1-(2,4-DICHLOROPHENYL)-1-[2-[(2-TETRAHYDROPYRANYL)OXY]METHYL]-PHENYL ETHANOL

Add 16g of crude oxirane of Example 1A in 25 mL of THF to a stirred suspension at 20° C of 5.1g of imidazolesodium (prepare by mixing 1.8g of NaH and 5.1g of imidazole at 5-20° C) in 75 mL of dimethylformamide (DMF). Heat the mixture for 2 hours on a steam bath. Pour the resulting mixture over water-ice and stir. Suction filter the mixture to leave a precipitate. Extract the filtrate with ethyl acetate (EtoAc). Wash the extract with brine and dry over anhydrous $Na_2SO_4$. Dry the precipitate in a vacuum desiccator at room temperature. Evaporate the EtOAc to give residue. Triturate the residue with EtOAc and combine the remaining solid with the dried precipitate and wash with EtOAc to leave 9.2g of crude 2-(1H-imidazol-1-yl)-1-(2,4-dichlorophenyl)-1-[2-[(2-tetrahydropyranyl)oxy] methyl]phenylethanol, which was used in the next step without further purification.

C. 1-(2,4-DICHLOROPHENYL)-1-(1H-IMIDAZOL-1-YLMETHYL)-1,3-DIHYDROISOBENZOFURAN HYDROCHLORIDE

Heat 0.8g of the crude alcohol from Example 1B at reflux in 30 mL of 6N hydrochloric acid for 1.5 hours. Pour the hot reaction mixture into a mixture of 50 mL of 3N NaOH, 50 mL of a 10% $K_2CO_3$ solution and 50g of ice. Extract the mixture with ethyl acetate. Dry the extract over anhydrous $Na_2SO_4$, filter and evaporate to a residue. Chromatograph the residue on silica gel eluting with $CH_2Cl_2$-EtOAc to afford 0.5g of a solid product. Dissolve the product in ethyl ether and treat the solution with dry HCl. Evaporate the resulting mixture to leave a solid. Recrystallize the solid from $CH_3CN$ to leave the title compound, m.p. 220-222° C.

EXAMPLE 2

1-(2,4-DIFLUOROPHENYL)-1-(1H-IMIDAZOL-1-YLMETHYL)-1,3-DIHYDROISOBENZOFURAN HYDROCHLORIDE

A. 1-(2,4-DIFLUOROPHENYL)-1[2-[(2-TETRAHYDROPYRANYL) OXY]-METHYL]PHENYL OXIRANE

Follow the procedure of Example 1A, except substitute 25.2g of 2-bromobenzyl-(2-tetrahydropyranyl) ether, 2.26g of magnesium, and 50 mL of THF in the first part; and substitute 16.9g 2,4-difluorophenacyl chloride, 400 mL of THF, and 200 mL of saturated aqueous $NH_4Cl$ in the second part. Obtain 33.5g of crude 1-(2,4-difluorophenyl)-1-[2-[(2-tetrahydropyranyl)oxy]methyl] phenyl oxirane.

B. 2-(1H-IMIDAZOL-1-YL)-1-(2,4-DIFLUOROPHENYL)-1-[2-[(2-TETRAHYDROPYRANYL)OXY]METHYL]-PHENYL(ETHANOL)

Follow the procedure of Example 1B, except substitute 33.5g of crude oxirane of Example 2A, 150 mL of THF, 12.0g of imidazolsodium, and 150 mL of DMF. Obtain 21.1g of crude 2-(1H-imidazol-1-yl)-1-(2,4-fluorophenyl)-1-[2-[(2-tetrahydropyranyl)oxy]methyl] phenylethanol.

C. 1-(2,4-DIFLUOROPHENYL)-1-(1H-IMIDAZOL-1-YLMETHYL)-1,3-DIHYDROISOBENZOFURAN HYDROCHLORIDE

Follow the procedure of Example 1C, except substitute 21.9g of the crude product of Example 2B and 300 mL of 6N hydrochloride acid in the first part; and substitute proportional amounts of 3N NaOH, 10% $K_2CO_3$ solution, and ice in the second part. Obtain after crystallization from $CH_3CN/Et_2O$, 6.65g of the title compound, m.p. 143-146° C.

## EXAMPLE 3

### 1-(4-CHLOROPHENYL)-1-(1H-IMIDAZOL-1-YLMETHYL)-1,3-DIHYDROISOBENZOFURAN HYDROCHLORIDE

A. 1-(4-Chlorophenyl)-1-[2-[(2-tetrahydropyranyl)oxy]-methyl]phenyl oxirane is prepared from 2-bromobenzyl-(2-tetrahydropyranyl)ether and 4-chlorophenacyl chloride in accordance with procedure of Example 1A.

$^1$H NMR ($CDCl_3$) δ: 1.3-2.0(m,6H), 3.1-3.8(m) and 3.3(q), [4H total], 4.5-4.8(m,3H), 7.0-7.9(m,8H).

B. 1-(4-Chlorophenyl)-2-(1H-imidazol-1-yl)-1-[2-[(2-tetrahydropyranyl)oxy]methyl]phenylethanol is prepared from the title compound of Example 3A in accordance with procedure of Example 1B.

$^1$H NMR ($CDCl_3$) δ: 1.3-2.0(m,6H), 3.2-3.8(m,2H), 4.1(q,2H), 4.3(m) and 4.8(q) [3H total], 5.0(s,1H), 6.5(s,1H), 6.7(s,1H), 7.0-8.0(m,8H).

C. 1-(4-Chlorophenyl)-1-(1H-imidazol-1-ylmethyl)-1,3-dihydroisobenzofuran hydrochloride, m.p. 192-193 °C is prepared from title compound of Example 3B in accordance with the procedure of Example 1C.

As previously mentioned the selected compounds of the general formula I have unexpectedly been found to possess valuable in vivo antifungal activity against human and animal pathogens including dermatophytes and strains of Candida. This finding may be illustrated by way of the in vitro and in vivo antifungal activity data presented in the following Table I for representative compounds of the present invention, that is Compounds 3, 4 and 5 of Table 1, and for compounds falling outside the scope of the present invention but within the scope of the compounds disclosed in British Patent Application 2,143,523A.

TABLE 1

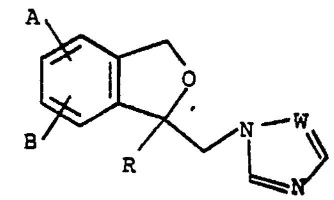

(X)

| Compound No. | W | A | B | R | Anti-fungal activity: in vitro MICs Dermat. | in vitro MICs Candida | in vivo Candida Test (% Inhibition) 0.01% | in vivo Candida Test (% Inhibition) 0.05% (Dose) |
|---|---|---|---|---|---|---|---|---|
| 1 | CH | 4-Cl | 6-Cl | 2,4-dichloro-phenyl | 9.5 | 2.0 | Nil | Nil |
| 2 | CH | H | 6-Cl | 2,4-dichloro-phenyl | < 0.38 | < 0.03 | 26 | 64 |
| 3 | CH | H | H | 4-chloro-phenyl | < 0.86 | < 0.015 | 30 | 88 |
| 4 | CH | H | H | 2,4-dichloro-phenyl | < 0.04 | < 0.015 | 78 | 94 |
| 5 | CH | H | H | 2,4-difluoro-phenyl | < 0.65 | < 0.015 | 69 | 88 |
| 6 | CH | H | H | phenyl | < 5.2 | 0.2 | N.T.* | N.T. |
| 7 | CH | H | H | biphenyl | < 0.12 | 0.16 | N.T. | N.T. |
| 8 | CH | H | H | 4-chloro-benzyl | 4.6 | 0.25 | N.T. | N.T. |

*N.T. indicates not tested (insufficiently active *in vitro*).

The *in vitro* activities given in Table I are Minimum Inhibitory Concentrations (MIC's) determined according to conventional micro-biological techniques using a broth dilution method as described in "Manual of Chemical Microbiology", American Society for Microbiology, E. H. Lennette, et al. (1980) pp. 648-649. The column in Table I headed "Dermat." lists the geometric mean MIC's (ug/ml; 72 hour values; 37° C) against 5 strains of dermatophytes assayed in Sabourand dextrose broth (pH5.7). The column headed "Candida" lists the geometric mean MIC's (ug/ml; 48 hour values; 37° C) against 6 strains of Candida assayed in Eagle's minimum essential medium (pH7.0).

The *in vivo* (topical) activities given in Table I were determined using the hamster vaginal *Candida* test described in "Abstracts, Interscience Conference in Antimicrobial Agents and Chemotherapy", 1982. The percent inhibition index at doses of 0.01 percent and 0.05 percent was determined by taking cultures on day 2, 4, 6, 8 and 11 following treatment and then scoring the cultures as follows:

4 = turbidity equal to control

3 = turbidity lighter than control

2 = no visible turbidity, but cells seen under microscope

1 = no cells seen under microscope, but plated out on agar from 0.2 ml aliquot

0 = agar plate negative

The group scores obtained for all five sampling periods (i.e. at days 2, 4, 6, 8 and 11) were totaled. The total was then multiplied by the number designating the last period (day 2, 4, 6, 8 and 11) to produce a positive culture. This number was then divided by the number similarly obtained from the solvent control and then substracted from 100 to give the percent inhibition of control.

As will be seen from Table 1, the selected compounds of the invention, Compounds 3, 4 and 5, which are halo-substituted in the phenyl group but unsubstituted in the aromatic ring of the isobenzofuran structure possess particularly good *in vivo* activity in the *Candida* test at one or both of doses 0.01% and

0.05% as compared to compounds which are halo substituted in both the phenyl group and the aromatic ring of the isobenzofuran structure (see, for instance, Compound 1 of Table 1 which was inactive in the Candida test). From a consideration of miconazole and related compounds it would be expected, however, that substitution in both the phenyl group and the aromatic ring of the isobenzofuran structure would be necessary and, indeed, essential to obtain high antifungal activity. However, as will be seen from Table 1, formation of the cyclized isobenzofuran structure corresponding to the open-ring structure of miconazole (1-[2-(2,4-dichlorophenyl)-2[(2,4-dichlorophenyl)methoxy]ethyl]-1H-imidazole) gives a product devoid of activity in the Candida test (see Compound 1 of Table 1).

As will also be seen from Table 1, compounds outside the small selected group of compounds of the present invention, but related thereto, are inactive or relatively inactive in vivo using the Candida test as the reference point. Thus, compounds unsubstituted in the phenyl moiety of the structure of the general formula X see Compounds 6 and 7) are insufficiently active in vitro (and hence were not tested in the Candida test). Further, replacement of the substituted phenyl group in the compounds of the general formula X by a substituted benzyl group leads to inactive products (see Compound 8).

For comparison, a triazole of the general formula X wherein W is nitrogen was compared with the corresponding imidazole of the present invention having W being $-CH_2$. (Compound 3 of Table 1 above.) The triazole was a preferred plant-growth regulating and plant antifungal compound disclosed in G.B. 2,143,523A, namely 1-(4-chlorophenyl)-1-(1,2,4-triazol-1-ylmethyl)-1,3-dihydroisobenzofuran the subject of Example 1 of G.B. 2,143,523A. The reference triazole was found to be toxic in mice, per oral, at a dose of 100 mpk. The corresponding Compound 3 of the invention was non-toxic at 100 mpk.

In general, the dosage of compounds of formula I administered to combat a given fungal infection will be similar to the dosage requirements of the present commercial products miconazole and clotrimazole.

In general, the topical dosage range will be from about 0.1% to about 10% by weight of a particular pharmaceutical composition formulated in single or divided doses, with the preferred range being about 0.5% to about 4% and with the most preferred range being about 1% to about 2%.

It will be appreciated that the actual preferred dosages of the compounds of this invention or pharmaceutically acceptable salts thereof will vary according to the particular compound being used, the particular composition formulated, the mode of application and the particular situs, host and disease being treated.

In another of its aspects the present invention provides pharmaceutical compositions, suitable especially for the treatment of human and animal fungal infections, comprising as active ingredient a compound of the general formula I hereinbefore set forth together with a pharmaceutically acceptable carrier. Preferred pharmaceutical composition are those adapted for topical and parenteral use.

Topical dosage forms may be prepared according to procedures well known in the art, and may contain a variety of ingredients. The formulations for topical use include ointments, creams, lotions, powders, aerosols, vaginal tablets, pessaries and sprays. Of these, ointments, lotions and creams may contain water, oils, fats, waxes, polyesters, alcohols, or polyols, plus such other ingredients as fragrances, emulsifiers and preservatives. Powders are made by mixing the active ingredient with a readily available, inert, pulverous distributing agent, such as talcum, calcium carbonate, tricalcium phosphate, or boric acid. Aqueous suspensions of the above powders may also be made. Solutions or emulsions may also be prepared using inert solvents which are preferably nonflammable, odorless, colorless and non-toxic, for example vegetable oils, isopropanol, dimethyl sulfoxide, hydrogenated naphthalenes, and alkylated naphthalenes. Similarly, aerosol or non-aerosol sprays may be prepared using solutions or suspensions in appropriate solvents, e.g., difluorodichloromethane for aerosols.

In the case of topical formulations, e.g., ointments, creams, lotions, powders, tablets, pessaries or sprays, the formulation will contain about 0.1 to 10 grams of a compound of formula I per 100 grams of carrier.

Parenteral forms to be injected intravenously, intramuscularly, or subcutaneously are usually in the form of a sterile solution, and may contain salts or glucose to make the solution isotonic.

The following Examples illustrate typical pharmaceutical formulations containing as the active ingredient (designated "Drug") a compound of this invention, such as 1-(2,4-difluorophenyl)-1-(1H-imidazol-1-ylmethyl)1,3-dihydroisobenzofuran.

## FORMULATION EXAMPLE 1

| Table 125.00 mg. tab. | |
|---|---|
| Drug | 125.00 mg. |
| Polyethylene glycol 6000 | 100.00 mg. |
| Sodium lauryl sulfate | 6.25 mg. |
| Corn starch | 30.00 mg. |
| Lactose, anhydrous | 87.25 mg. |
| Magnesium stearate | 1.50 mg. |

Procedure:

Heat the polyethylene glycol 6000 to 70-80 °C. Mix the drug, sodium lauryl sulfate, corn starch, and lactose into the liquid and allow the mixture to cool. Pass the solidified mixture through a mill. Blend granules with magnesium stearate and compress into tablets.

FORMULATION EXAMPLE 2

| Capsule 250 mg. tab. | |
|---|---|
| Drug | 250.00 mg. |
| Lactose, anhydrous | 100.00 mg. |
| Corn starch | 50.00 mg. |
| Microcrystalline cellulose | 95.00 mg. |
| Magnesium stearate | 5.00 mg. |

Procedure:

Mix the first four ingredients in a suitable mixer for 10-15 minutes. Add the magnesium stearate and mix for 1-3 minutes. Fill the mixture into suitable two-piece hard gelatin capsules using an encapsulating machine.

**Claims**

1. A compound of the general formula I:

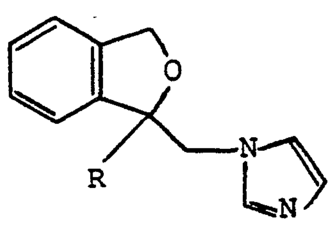

(I),

and the pharmaceutically acceptable salts thereof, wherein R represents a halo- and/or trifluoromethyl-substituted phenyl group.

2. A compound as claimed in claim 1, wherein R represents a chloro- or fluoro-substituted phenyl group.

3. A compound is claimed in claim 1 or claim 2, wherein R represents monohalophenyl, or more preferably dihalophenyl.

4. A compound as claimed in any one of the preceding claims where R represents 2,4-dichlorophenyl or 2,4-difluorophenyl.

5. A compound as claimed in claim 1, said compound being selected from:
1-(2,4-dichlorophenyl)-1-(1H-imidazol-1-ylmethyl)-1,3-dihydroisobenzofuran, and
1-(2,4-difluorophenyl)-1-(1H-imidazol-1-ylmethyl)-1,3-dihydroisobenzofuran.

6. A process for the preparation of a compound of the formula I set forth in claim 1, which process comprises a process selected from the following processes A and B:

A. cyclizing a compound of the formula II,

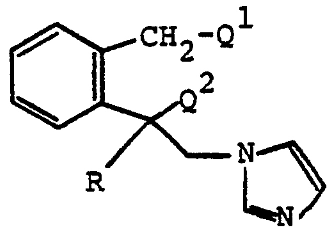

(II),

wherein R is as defined in claim 1 and of $Q^1$ and $Q^2$ one is hydroxy and the other is hydroxy, halo or a reactive ether or ester group, in the presence of a solvent and a cyclizing agent and at a temperature up to the reflux temperature of the reaction mixture, and

B. reacting a compound of the general formula III:

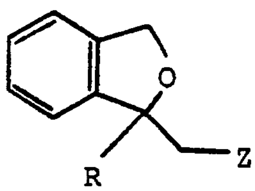

(III),

wherein R is as defined in claim 1 and Z is halo or is a reactive ester group, with imidazole in the presence of a strong base, or with an alkali metal derivative of imidazole, in an aprotic organic solvent at a temperature up to the reflux temperature of the reaction mixture, the so-obtained compound of the general formula I being isolated in free form or in the form of a pharmaceutically acceptable salt.

7. A pharmaceutical composition comprising as active ingredient a compound as claimed in any one of claims 1 to 5 together with a pharmaceutically acceptable carrier.

8. A pharmaceutical composition as claimed in claim 7, said composition being in dosage form.

9. The use of a compound as claimed in any one of claims 1 to 5 for the preparation of a pharmaceutical composition for use as an antifungal agent.

European Patent Office

# EUROPEAN SEARCH REPORT

Application Number

EP 88 30 3239

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| X | US-A-4 737 508 (R.G. LOVEY)<br>* Columns 3-6; claims *<br>--- | 1-9 | C 07 D 405/06<br>A 61 K 31/415//<br>C 07 D 407/12<br>C 07 D 405/12 |
| D,A | GB-A-2 143 523 (I.C.I.)<br>* Claims *<br>----- | 1,6 | |
| | | | **TECHNICAL FIELDS SEARCHED (Int. Cl.4)** |
| | | | C 07 D 405/00<br>C 07 D 233/00<br>C 07 D 249/00<br>A 61 K 31/00 |

The present search report has been drawn up for all claims

| Place of search | Date of completion of the search | Examiner |
| --- | --- | --- |
| THE HAGUE | 08-11-1988 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................
& : member of the same patent family, corresponding document

EPO FORM 1503 03.82 (P0401)